# EUROPEAN PATENT SPECIFICATION

(11) **EP 4 751 668 B1**
(45) Date of publication and mention of the grant of the patent: **04.11.2026**
(21) Application number: 24461641.3
(22) Date of filing: 28.11.2024
(51) Int. Cl.: A61B 17/80

(54) **PECTUS BAR SYSTEM**
BRUSTSTANGENSYSTEM
SYSTÈME DE BARRE DE PECTUS

(43) Date of publication of application: 03.06.2026
(73) Proprietor: CHM SPOLKA Z OGRANICZONA ODPOWIEDZIALNOSCIA, 16-061 Lewickie (PL)
(72) Inventor: KRZYZEK Andrzej, 15-666 Bialystok (PL); ZDANOWICZ Ruslan, 15-460 Bialystok (PL); MURAWSKI Zbigniew, 15-034 Bialystok (PL); ZACHA Slawomir, 72-003 Dobra (PL)
(74) Representative: Bury & Bury

(56) References cited:
- CN-U- 202 458 636
- CN-U- 219 782 706
- US-A1- 2004 117 016
- US-A1- 2010 256 691
- US-A1- 2015 134 009

## Description

### Field of the invention

The invention concerns a pectus bar system, in particular for the treatment of chest deformities.

### State of the art

Pectus bar system (or sterno-costal plate system) plates are used in the treatment of chest deformities, the so-called funnel chest *(pectus excavatum)* using Dr. Nuss's Minimally Invasive Repair of *Pectus Excavatum* (MIRPE) method, which involves inserting a correction plate under the sternum to correct the deformity. Pectus bar systems are available on the market. They are offered by ChM sp. z o.o. together with the instrumentation 40.5841.000. The description of the surgical technique was published by the manufacturer in 2012 under the number ST/42A.

During the operation, the patient is placed supine with both arms abducted at the shoulders. A sterile marker is used to mark the lowest point of the funnel chest or, if this point is below the sternum, the lowest point of the sternum. On both sides of the chest, intercostal spaces are marked in line with the marked point on the sternum or passing closest to the marked point. On the extension of the marked line, between the anterior axillary line and the middle axillary line, a transverse incision is made for inserting the correction plate. The selection of the length of the correction plate should be preceded by appropriate measurements. The shape and length of the plate depend on the shape of the deformation. The correct shaping of the plate is carried out by bending it using a bending tool available in the instrumentation.

During the insertion of the plate of the pectus bar system as an implant, it is necessary to avoid forming sharp arcs, over-bending, and bending the implant in places where the holes are located. There is a certain risk of displacement or rotation of the implant. This may consequently lead to damage to tissues or organs located near the plate. During the implantation procedure, special care should be taken to avoid contact of the implant or instruments with the heart and lungs. In this connection, stabilizing plates are used that are connected during the operation to the correction plate. The stabilizing plate is put on the end of the correction plate of pectus bar system and attached to the chest muscles. The stabilizing plates are provided with sockets for receiving the correction plate, preventing the rotation of the correction plate relative to the stabilizing plate, and with holes for connecting the correction and stabilizing plates by screwing.

Document KR20130045009A discloses a pectus bar system comprising a correction plate which is connected to the stabilizing plates by a screw passing through a hole in the corrective plate and screwed into the correction plate.

US2011251540A1 discloses a device and method for treating pectus excavatum. The device includes a bar stabilizer assembly having two or more channels for securing correction plates (pectus bars) and retaining them in the patient's chest cavity. The correction plates are connected to the stabilizing plates by placing them in sockets and locking them with a screw passing through one of the holes provided in the correction plate and screwed into the stabilizing plate. This approach involves some complication of the surgical technique, since it is necessary to place the end of the correction plate in the channel of the stabilizing plates during the operation and also to adjust the position of the two holes and the screw.

European patent EP3579772B1 discloses a pectus bar system comprising a correction plate and a stabilizing plate. The stabilizing plate has a channel that is shaped to fit the correction plate and is adapted to receive the correction plate and allow it to move. A mechanism is provided in this channel to prevent the movement the correction plate by rotating the key of the cam provided.

A further pectus bar system is known from US 2015/134009 A1.

### Problem to be solved

The purpose of the invention is to provide a pectus bar system that simplifies the surgical technique, shortens the time needed to insert the correction plate into the chest cavity and reduces the risk to which the patient is exposed. An additional purpose is to simplify the complex manufacturing process and increase the tolerance of the components required for safe mounting.

### Summary of the invention

The pectus bar system according to the invention comprises at least one correction plate of elongated shape having narrowed sections with rows of holes at its ends and at least one stabilizing plate with rows of holes adapted to be attached to the side of the patient's chest. The stabilizing plate and the correction plate are adapted to be connected to each other by a screw connection through said holes. The holes of the correction plate are provided with internal threads. The system according to the invention further comprises a headless double-threaded positioning screw provided with a socket for a tool and having a first thread complementary to the thread of the hole in the correction plate. The positioning screw is also provided with a second thread that can pass through (fits loosely) the hole in the stabilizing plate. The system also comprises a nut provided with an internal thread complementary to the second thread of the positioning screw, the nut having a head wider than the hole in the stabilizing plate. The correction plate is provided with toothing around the holes, whereas the stabilizing plate is provided with toothing complementary to the toothing of the correction plate around the holes, around at least part of their circumference. This provides a stable connection of the plates, which can be obtained in a simple and predictable surgical implantation process. This process requires the system of only two, and not three or more elements, in each step.

The system preferably comprises two stabilizing plates.

Preferably, the toothing of the holes in the correction plate is concave and the toothing of the holes in the stabilizing plate is convex (raised).

Preferably, the toothing of the holes in the stabilizing plate covers only sections of the circumference of the holes arranged substantially parallel to the length of the stabilizing plate. The nut is preferably provided with a shank accommodating at least partially in the hole in the stabilizing plate. Such a configuration provides a sufficiently long thread section without excessively increasing the height of the connection.

Preferably, the positioning screw is provided with a collar between the first thread and the second thread with a diameter larger than the smallest diameter of the hole in the correction plate. Such a solution enables the screwed screw to be locked at the correct depth.

Preferably, the collar of the screw has a smaller diameter than the hole in the stabilizing plate, which enables the nut shank to contact the correction plate and, consequently, better stabilization.

Preferably, the collar of the screw is conical and the hole in the correction plate has a complementary conical shape in its upper part. This ensures better locking.

Preferably, the positioning screw has an unthreaded section at the end opposite the socket, which makes it easier to insert it into the hole.

### Advantageous results of the invention

Complementary toothing of the holes in the correction plate and in the stabilizing plate prevents the plates from rotating with respect to each other even at a relatively low torque of tightening the nut. Moreover, after putting on the nut and before tightening it, these elements can be rotated relative to each other without disconnecting them, which facilitates the installation of the stabilizing plate. The use of toothed surfaces to prevent the rotation of the plates, instead of channels in the stabilizing plates known in the art, simplifies the process of connecting the correction and stabilizing plates and allows one to avoid the need to put the stabilizing plates on the ends of the correction plate, which putting can be tedious, especially if the correction plate required bending. The system according to the invention makes it possible to connect two (not three) elements at once during the operation and gradually obtain the connection of three elements. Owing to this, troublesome simultaneous aligning two holes and a screw is avoided. After bending the correction plate, it is easy to select the correct hole into which the positioning screw will be screwed and, before the operation, check, by screwing in the positioning screw, whether the hole has not been deformed due to bending. Owing to this, the risk to which the patient is exposed is reduced. The system allows one to apply subsequent elements from the outside and does not require access from below, i.e., from the patient's interior. This reduces the invasiveness of the procedure.

### Description of the drawing

The invention is described in detail below presenting embodiments in reference to the attached drawings.
Fig. 1 schematically shows an exemplary pectus bar system mounted on a patient.
Fig. 2 shows a method of connecting a correction plate to a stabilizing plate using a positioning screw and a nut in an embodiment of the invention.
Fig. 3 shows a correction plate according to an embodiment of the invention, in a top view, i.e. from the surface facing away from the patient's body, after mounting.
Fig. 4 shows a correction plate according to an embodiment of the invention, in a bottom view, i.e. from the bottom side facing the patient's body, after mounting.
Fig. 5 shows the end part of the correction plate according to an embodiment of the invention in an enlarged and perspective view showing the toothing around the holes.
Fig. 6 schematically shows a correction plate according to an embodiment of the invention, in a side view with the cross-section of the holes highlighted.
Fig. 7 shows a stabilizing plate according to an embodiment of the invention, in a bottom view, i.e. from the bottom facing the patient's body after mounting.
Fig. 8 shows a stabilizing plate according to an embodiment of the invention, in a top view, i.e. from the top side facing away from the patient's body after mounting.
Fig. 9 shows a stabilizing plate according to an embodiment of the invention, in a perspective view, enlarged, showing the last two holes at the end of the plate.
Fig. 10 shows a stabilizing plate according to an embodiment of the invention, in a side view, enlarged, showing the last two holes at the end of the plate, with the cross-section of the holes highlighted.
Figs. 11 and 12 show a positioning screw according to an embodiment of the invention.
Figs. 13 and 14 show a nut according to an embodiment of the invention.

### Embodiments of the invention

The pectus bar system is used by inserting at least one correction plate **10** under the patient's sternum and stabilizing it with at least one, but preferably two, stabilizing plates **30.** The stabilizing plate **30** is optionally attached to the patient's costae or to his muscles so as to hold the correction plate **10** inserted under the sternum and ensure its stable position. The stabilizing plates **30** prevent the correction plate **10** from rotating. Sometimes one stabilizing plate **30** is used, and in other cases two. In some cases it is also justified to use two correction plates **10.** The system according to the invention provides the possibility of using all of these configurations.

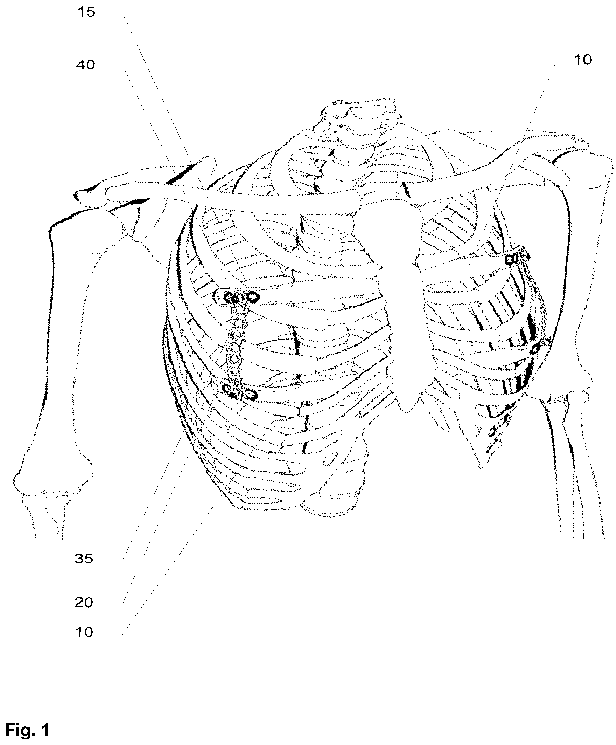

The pectus bar system according to an embodiment of the invention is shown together with the patient's skeleton in Fig. 1. In this embodiment, the system comprises two stabilizing plates **30** and two correction plates **10** forming a stable frame. Owing to the solution according to the invention, the possibility of "tilting" the attached system is limited, because the connections of the stabilizing plates **30** and the correction plates **10** are secured against rotation. Therefore, there is no need to attach the stabilizing plates to the patient's costae or to his muscles.

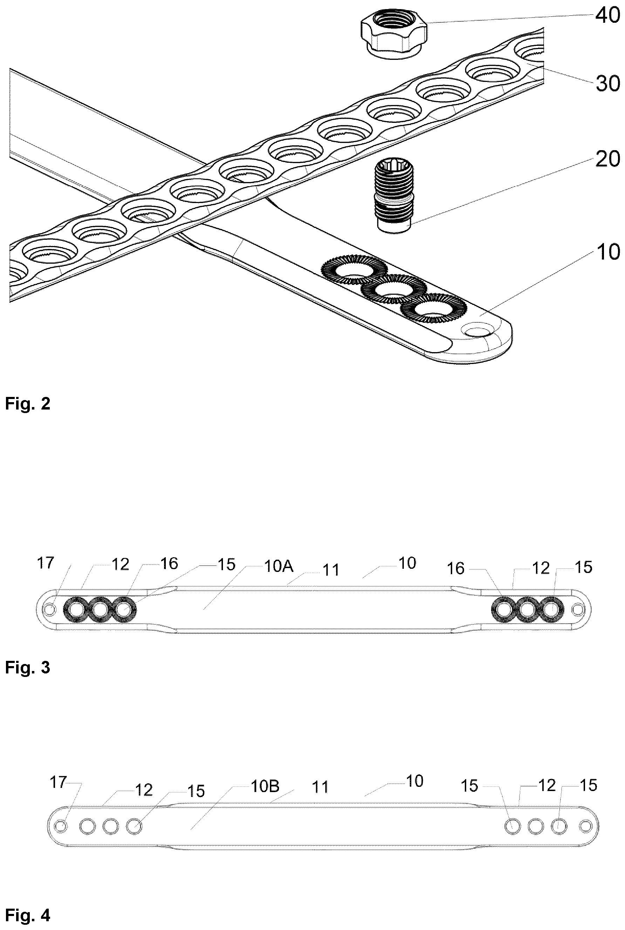

A stable connection of the correction plate **10** and the stabilizing plate **30** in the system according to the embodiment of the invention is made in the manner illustrated in Fig. 2.

A screw **20** is screwed into the threaded hole **15** of the correction plate 10 using a screwdriver with a tip that fits into the screw's socket **29.**

When the patient lies on his back, the correction plate **10** is oriented with its top side **10A** upwards, away from the patient's body, and with its bottom side **10B** downwards, towards the patient's body. Since the hole **15** is threaded, screwing in the positioning screw **20** does not require access to the correction plate **10** from below since access from above is sufficient. This minimizes the need to move the plate **10** in the patient's body. The screwing step requires the alignment of only two elements: the positioning screw **20** and the correction plate **10.**

A typical problem associated with bending the correction plates known in the art is the deformation of the hole in the correction plate during its adaptation to the patient's needs. This risk also occurs in the system according to the invention, but it is easy to try on the correction plate on the patient before the procedure and check the alignment of the positioning screw **20** and the target hole **15.** Screwing problems can therefore be detected before an actual surgical operation starts and, if they occur, a new correction plate can be bent and the damaged one rejected. In this way, the risky alignment of several elements of the system during the operation is avoided.

The hole **35** in the stabilizing plate **30** is put on the positioning screw **20** screwed into the correction plate. This can be done quickly and without problems, because the holes **35** are not threaded. The stabilizing plate **30** is oriented with the bottom side **30A** downwards, i.e. so that the bottom side **30A** faces the patient's body and the correction plate **10** which is oriented with the top side **10A** upwards. After the hole **35** in the stabilizing plate **30** is put on the positioning screw **20,** a nut **40** is screwed onto the positioning screw **20** behind the stabilizing plate **30.** Until the nut **40** is tightened, the stabilizing plate **30** can be rotated relative to the correction plate **10,** but they remain connected and the system does not fall apart. Owing to this, the stabilizing plate is easy to attach. Slightly tightening the nut results in a slight resistance to rotation, which eliminates accidental displacements. After attaching the stabilizing plate **30,** the nut **40** is tightened. Owing to the complementary toothing **16, 36** of the holes **15, 35,** the plates are locked with a tightening torque which is relatively low, which eliminates a risk of so-called cold welding and makes it much easier and safer to disassembly the pectus bar system remove the correction plates **10** when the therapy is ended.

The correction plate **10** according to an embodiment of the invention is illustrated in Fig. 3-6. The correction plate **10** is made in variants having a length of 180÷405 mm and a thickness of 2.8 mm. In the central portion **11,** the correction plate **10** has a width of 14 mm. In the end portion **12,** the correction plate **10** narrows to a width of 12 mm. At the ends of the correction plate **10,** holes **17** with a diameter of 3 mm are provided to facilitate inserting the correction plate **10** under the sternum. These holes also make it easier to hold the correction plate using an auxiliary hook during attachment of the stabilizing plates **30.** A person skilled in the art may select other plate lengths and thicknesses or hole diameters if necessary. However, the combination indicated above has proven to be convenient and compatible both with the dimensions of the patients and with the available instruments.

In the present embodiment, the correction plate **10** is made of a titanium alloy that provides an appropriate flexibility. Ti6Al4V is particularly advantageous. Alternatively, the plates can also be made of pure titanium, which is more ductile and bends more easily.

On the top side **10A,** facing away from the patient, the correction plate **10** has more bevelled edges than on the bottom side **10B** facing the patient. The bevel of the upper edge on the top side **10A** ensures compatibility with stabilizing plates known in the art and available, which are intended to be put onto the correction plate. This enables more efficient use of hospital storage resources, and the use of a convenient screwed connection, at least on one end, constitutes a significant simplification of the operation.

In the present embodiment, each of the end portions **12** of the correction plate **10** is provided with a row of four holes **15** for connection to the stabilizing plate **30.** It has been found experimentally that four holes are usually sufficient for use in patients with a wide range of chest sizes. The plates of pectus bar system are used in the treatment of *pectus excavatum* in patients aged 12-16 years.

In the present embodiment, holes **15** with a diameter of 4.7 mm (largest diameter of the cone) with an internal metric double thread of 4.5 mm are used. The holes **15** have a threaded cylindrical central part and a conical part widening towards the top side **10A,** i.e. away from the patient. In this conical part, a collar **22** of the positioning screw **20** is partially accommodated and locked after the screw is screwed in. Owing to such a design, the risk of accidental unscrewing is advantageously reduced and a more stable locking of the screw is obtained. Moreover, the conical widened hole makes it easier to insert the screw during the operation, thus reducing a risk of the operator dropping the screw decreases and the operation statistically takes less time.

On the top side **10A** of the correction plate **10** around the holes **15,** toothing **16** is provided along their entire circumference. In the present embodiment, this toothing **16** is provided along the entire circumference and is concave, i.e., the teeth are recesses in the top side **10A** of the correction plate **10.** These recesses are complementary to the convex toothing of the stabilizing plate **30.** Other types of complementary toothing can be used. It should be noted, however, that the use of concave toothing prevents the difficulties in inserting the correction plate under the sternum, which could occur with convex toothing.

In the present embodiment, toothing consisting of 50 teeth with a depth of 0.2 mm was used, which proved to be a good compromise between ease of manufacture and effectiveness and comfort of use. Effective connections and the convenient surgical insertion were achieved with toothing having from 18 to 100 teeth with a depth of from 0.1 mm to 0.5 mm.

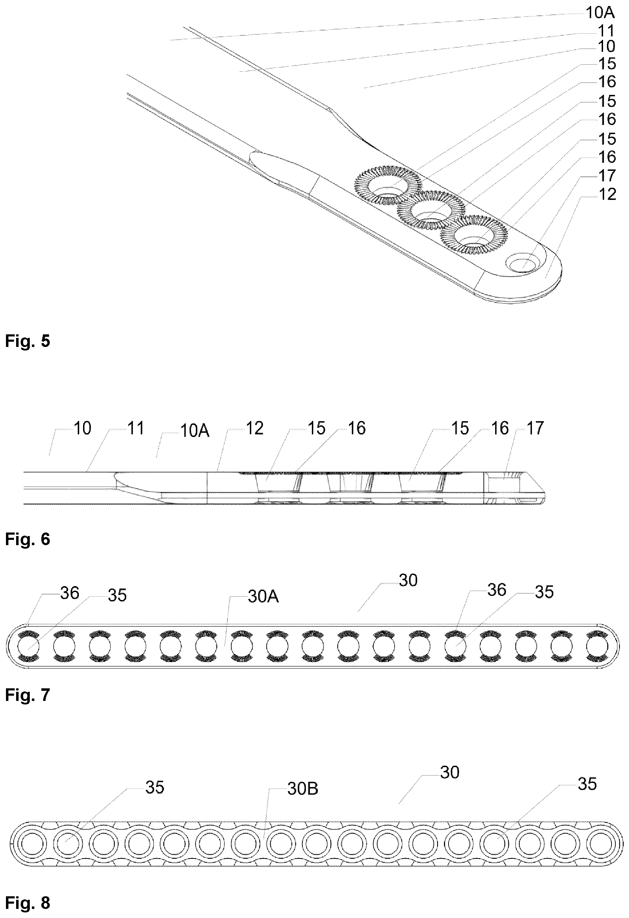

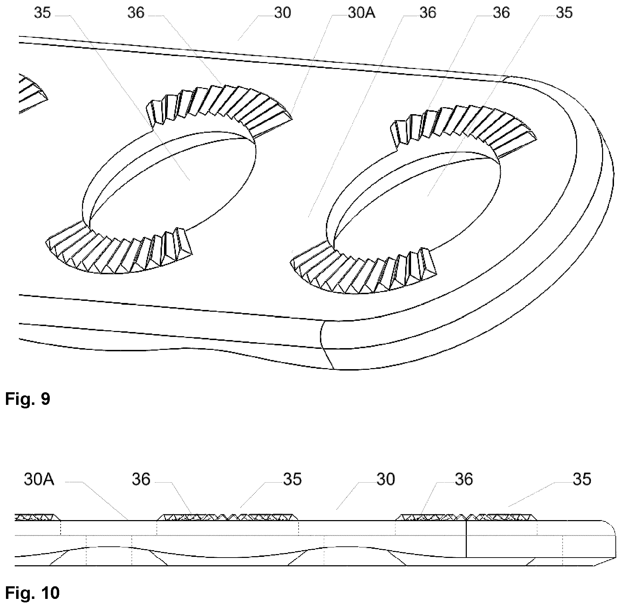

A stabilizing plate **30** according to an embodiment of the invention is shown in Figs. 7 - 10. In this embodiment, the stabilizing plate **30** has an elongated shape and includes 17 holes **35.** The width of the stabilizing plate **30** at the location of the holes **35** is greater due to the beveling of its edges, which can be seen in Figs. 7, 8, 9. The wavy bevel softens the edge and reduces irritation of the tissues surrounding the implant. The reduced bevel at the location of the holes improves strength.

The stabilizing plate **30** is made in variants having a length of 42÷186 mm, a thickness of 1.5 mm and a width of 10 mm. The holes **35** have a diameter of 4.8 mm and a countersink with a diameter of 6.5 mm. A thickness of 1.5 mm has proven to be optimal, but good effects have been obtained in a wider range, namely from 1 to 2 mm.

In the present embodiment, the stabilizing plate **30** is made of pure titanium. Plates can also be made of titanium alloys, but pure titanium is more ductile and bends more easily.

In the stabilizing plate **30,** on the bottom side **30A** facing the patient and the correction plate **10,** around the holes **35** on part of their circumference, convex toothing **36** is provided, protruding above the bottom side **30A.** The toothing **36** is provided only on parts of the circumference of the holes **35** running along the long outer edge of the stabilizing plate. It turns out that such toothing is sufficient and is significantly easier to produce than convex toothing around the entire circumference, and furthermore, toothing only on part of the circumference reduces tissue irritation when the stabilizing plate is inserting subcutaneously.

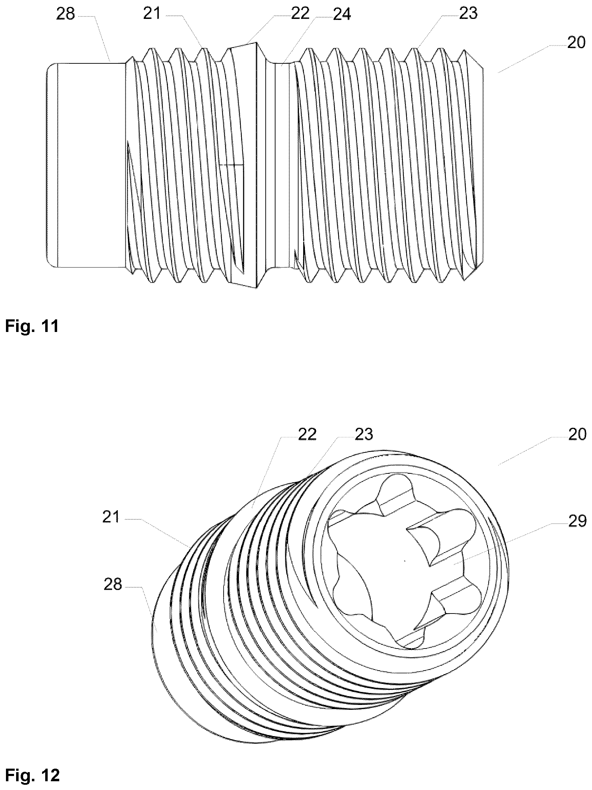

A positioning screw **20** according to an embodiment of the invention is shown in Figs. 11 and 12. The screw is provided with a first thread **21** complementary to the thread in the holes **15** in the correction plate **10** and a second thread **23** complementary to the thread **43** of the nut **40** and passing freely through the holes **35** in the stabilizing plate **30.** These threads are advantageously separated by a conically shaped collar **22.** Such a collar of the screw, matching the upper part of the hole **15** in the correction plate **10,** improves the hold of the tightened positioning screw **20.** The positioning screw **20** has no head, so that it passes through the holes **35** and fits into them without excessive backlash, but in the upper part it is provided with a socket **29** for a tightening tool, preferably a torx wrench. In the lower part, the screw **20** is advantageously provided with an unthreaded section **28** which makes it is easier to place the positioning screw **20** in the hole **15** straight, axially with respect to the internal thread. Owing to this unthreaded section **28,** the positioning screw **20** has a clearer asymmetry and the operator can assess its orientation more quickly. The section **24** visible in Fig. 11 is a technological undercut for the thread **23.**

In the present embodiment of the invention, the positioning screws **20** are made of a titanium alloy and have a diameter of 4.7 mm (the largest diameter of the cone). The first thread has a diameter of 4.5 mm, a length of 2 mm and a pitch of 1 mm. The second thread has a diameter of 4.5 mm, a length of 3.5 mm and a pitch of 1 mm. In the present embodiment, both threads are identical and both are double. This is technologically simple, but different threads can also be used. Incompatibility of the second thread **23** with the thread of the hole **15** additionally protects against mistakes during the operation.

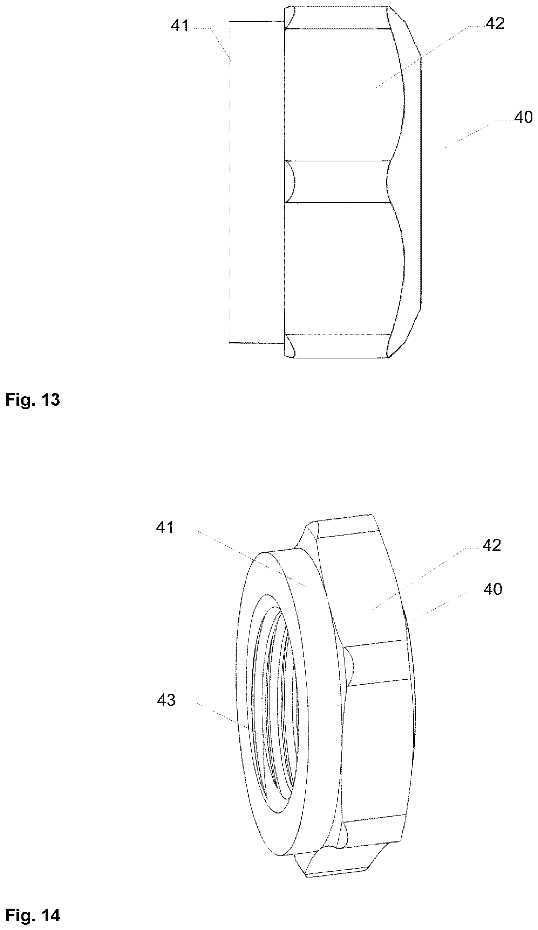

The nut **40** is shown in Figs. 13 and 14. The nut **40** has an internal thread **43** complementary to the second thread **23** of the positioning screw **20** and a head **42** fitting the tip of a tightening tool, in particular a torque wrench. Advantageously, the nut **40** is provided with a shank **41** that at least partially accommodates in the hole **35** of the stabilizing plate **30.** Such a solution stabilizes the connection of the correction plate **10** and the stabilizing plate **30.** The total height of the connection is also reduced and, consequently, irritation of the tissues surrounding the implant is reduced, because the nut is partially sunk in the hole in the plate while maintaining the necessary thread height.

### Industrial applicability of the invention

The invention provides of quick and reliable connection of correction plates and stabilizing plates in the process of treating pectus excavatum. The system according to the invention shortens the operation time, reduce its invasiveness and the risk to which the patient is exposed. The elements of the system are designed to eliminate particularly troublesome or error-prone actions from the operation, in particular connecting three elements at once or pressing elongated elements through channels. The system is selected so that the operation of inserting the correction plate is carried out "from the bottom up", without the need to operate under the correction plate in later stages of the operation. The system can be used with a different number of individual elements, which can be supplied together or separately.

## Claims

1. A pectus bar system comprising
at least one correction plate **(10)** of elongated shape having narrowed end portions **(12)** with rows of holes **(15),**
at least one stabilizing plate **(30)** with rows of holes **(35),** the plate being adapted to be attached to the side of the patient's chest,
wherein the stabilizing plate **(30)** and the correction plate **(10)** are adapted to be connected to each other by a screw connection through the holes **(15, 35)**
**characterized in that**
the holes **(15)** of the correction plate **(10)** are provided with internal threads, while
the system further comprises
a headless double-threaded positioning screw **(20)** provided with a socket **(29)** for a tool and having a first thread **(21)** complementary to the thread of the hole **(15)** in the correction plate **(10),**
wherein the positioning screw **(20)** is also provided with a second thread **(23)** passing through the hole **(35)** in the stabilizing plate **(30),** and
a nut **(40)** provided with an internal thread **(43)** complementary to the second thread **(23)** of the positioning screw **(20),** said nut having a head **(42)** wider than the hole **(35)** in the stabilizing plate **(30),**
wherein the correction plate **(10)** is provided with toothing **(16)** around the holes **(15),** whereas the stabilizing plate **(30)** is provided with toothing **(36)** complementary to the toothing **(16)** of the correction plate **(10)** located around the holes **(35),** on at least part of their circumference.

2. The system according to claim 1 having at least two stabilizing plates **(30).**

3. The system according to claim 1 or 2, wherein the toothing **(16)** of the holes **(15)** in the correction plate **(10)** is concave and the toothing **(36)** of the holes **(35)** in the stabilizing plate **(30)** is convex.

4. The system according to claim 3 wherein the toothing **(36)** of the holes **(35)** in the stabilizing plate **(30)** covers only sections of the circumference of the holes **(35)** arranged substantially parallel to the length of the stabilizing plate **(30).**

5. The system according to any one of claims 1 to 4 wherein the nut **(40)** is provided with a shank **(41)** accommodating at least partially in the hole **(35)** in the stabilizing plate **(30).**

6. The system according to any one of claims from 1 to 5 wherein the positioning screw **(20)** is provided with a collar **(22)** located between the first thread **(21)** and the second thread **(23),** having a diameter larger than the smallest diameter of the hole **(15)** in the correction plate **(10).**

7. The system according to claim 6 wherein the collar **(22)** has a smaller diameter than the hole **(35)** in the stabilizing plate **(30).**

8. The system according to claim 6 or 7 wherein the collar **(22)** is conical and the hole **(15)** in the correction plate **(10)** has a complementary conical shape in its upper part.

9. The system according to claim 6 or 7 or 8 wherein the positioning screw **(20)** has an unthreaded section **(28)** at the end opposite the socket **(29).**

## Patentansprüche

1. Pektusbügelsystem, umfassend
zumindest eine Korrekturplatte (10) von länglicher Form, die verschmälerte Endabschnitte (12) mit Reihen von Löchern (15) aufweist,
zumindest eine Stabilisierungsplatte (30) mit Reihen von Löchern (35), wobei die Platte dazu ausgebildet ist, an der Seite des Brustkorbs des Patienten befestigt zu werden,
wobei die Stabilisierungsplatte (30) und die Korrekturplatte (10) dazu ausgebildet sind, mittels einer Schraubverbindung durch die Löcher (15, 35) miteinander verbunden zu werden,
**dadurch gekennzeichnet, dass**
die Löcher (15) der Korrekturplatte (10) mit Innengewinden versehen sind, während das System ferner Folgendes umfasst:
eine kopflose Positionierschraube (20) mit zwei Gewinden, die mit einer Aufnahme (29) für ein Werkzeug versehen ist und ein erstes Gewinde (21) aufweist, das zu dem Gewinde des Lochs (15) in der Korrekturplatte (10) komplementär ist,
wobei die Positionierschraube (20) auch mit einem zweiten Gewinde (23) versehen ist, das durch das Loch (35) in der Stabilisierungsplatte (30) hindurchgeht, und
eine Mutter (40), die mit einem Innengewinde (43) versehen ist, das zu dem zweiten Gewinde (23) der Positionierschraube (20) komplementär ist, wobei diese Mutter einen Kopf (42) aufweist, der breiter als das Loch (35) in der Stabilisierungsplatte (30) ist,
wobei die Korrekturplatte (10) mit einer Verzahnung (16) um die Löcher (15) herum versehen ist, wohingegen die Stabilisierungsplatte (30) mit einer zu der Verzahnung (16) der Korrekturplatte (10) komplementären Verzahnung (36) versehen ist, die um die Löcher (35) herum an zumindest einem Teil ihres Umfangs angeordnet ist.

2. System nach Anspruch 1, das zumindest zwei Stabilisierungsplatten (30) aufweist.

3. System nach Anspruch 1 oder 2, wobei die Verzahnung (16) der Löcher (15) in der Korrekturplatte (10) konkav ist und die Verzahnung (36) der Löcher (35) in der Stabilisierungsplatte (30) konvex ist.

4. System nach Anspruch 3, wobei die Verzahnung (36) der Löcher (35) in der Stabilisierungsplatte (30) nur Abschnitte des Umfangs der Löcher (35) bedeckt, die im Wesentlichen parallel zur Längsrichtung der Stabilisierungsplatte (30) angeordnet sind.

5. System nach einem der Ansprüche 1 bis 4, wobei die Mutter (40) mit einem Schaft (41) versehen ist, der zumindest teilweise in dem Loch (35) in der Stabilisierungsplatte (30) aufgenommen ist.

6. System nach einem der Ansprüche 1 bis 5, wobei die Positionierschraube (20) mit einem Bund (22) versehen ist, der zwischen dem ersten Gewinde (21) und dem zweiten Gewinde (23) angeordnet ist und einen Durchmesser aufweist, der größer als der kleinste Durchmesser des Lochs (15) in der Korrekturplatte (10) ist.

7. System nach Anspruch 6, wobei der Bund (22) einen kleineren Durchmesser als das Loch (35) in der Stabilisierungsplatte (30) aufweist.

8. System nach Anspruch 6 oder 7, wobei der Bund (22) konisch ist und das Loch (15) in der Korrekturplatte (10) in seinem oberen Teil eine komplementäre konische Form aufweist.

9. System nach Anspruch 6 oder 7 oder 8, wobei die Positionierschraube (20) an dem der Aufnahme (29) gegenüberliegenden Ende einen gewindefreien Abschnitt (28) aufweist.

## Revendications

1. Système de barre de pectus comprenant
au moins une plaque de correction (10) de forme allongée ayant des parties d'extrémité rétrécies (12) dotées d'une rangée de trous (15),
au moins une plaque stabilisatrice (30) dotée d'une rangée de trous (35), la plaque étant conçue pour être fixée sur le côté du torse du patient,
dans lequel la plaque stabilisatrice (30) et la plaque de correction (10) sont conçues pour être reliées l'une à l'autre par une liaison par vis à travers les trous (15, 35)
**caractérisé en ce que**
les trous (15) de la plaque de correction (10) sont munis de filetages internes,
tandis que le système comprend en outre
une vis de positionnement (20) sans tête à double filetage munie d'une empreinte (29) pour un outil et ayant un premier filetage (21) complémentaire au filetage du trou (15) dans la plaque de correction (10),
dans lequel la vis de positionnement (20) est également munie d'un second filetage (23) passant à travers le trou (35) dans la plaque stabilisatrice (30), et
un écrou (40) muni d'un filetage interne (43) complémentaire au second filetage (23) de la vis de positionnement (20), ledit écrou ayant une tête (42) plus large que le trou (35) dans la plaque stabilisatrice (30),
dans lequel la plaque de correction (10) est munie d'une denture (16) autour des trous (15), alors que la plaque stabilisatrice (30) est munie d'une denture (36) complémentaire à la denture (16) de la plaque de correction (10) située autour des trous (35), sur au moins une partie de leur circonférence.

2. Système selon la revendication 1 ayant au moins deux plaques stabilisatrices (30).

3. Système selon la revendication 1 ou 2, dans lequel la denture (16) des trous (15) dans la plaque de correction (10) est concave et la denture (36) des trous (35) dans la plaque stabilisatrice (30) est convexe.

4. Système selon la revendication 3 dans lequel la denture (36) des trous (35) dans la plaque stabilisatrice (30) recouvre uniquement des sections de la circonférence des trous (35) agencées de manière sensiblement parallèle à la longueur de la plaque stabilisatrice (30).

5. Système selon l'une quelconque des revendications 1 à 4 dans lequel l'écrou (40) est muni d'une tige (41) se logeant au moins partiellement dans le trou (35) dans la plaque stabilisatrice (30).

6. Système selon l'une quelconque des revendications 1 à 5 dans lequel la vis de positionnement (20) est munie d'une collerette (22) située entre le premier filetage (21) et le second filetage (23), ayant un diamètre plus grand que le plus petit diamètre du trou (15) dans la plaque de correction (10).

7. Système selon la revendication 6 dans lequel la collerette (22) présente un plus petit diamètre que le trou (35) dans la plaque stabilisatrice (30).

8. Système selon la revendication 6 ou 7 dans lequel la collerette (22) est conique et le trou (15) dans la plaque de correction (10) présente une forme conique complémentaire dans sa partie supérieure.

9. Système selon la revendication 6 ou 7 ou 8 dans lequel la vis de positionnement (20) présente une section non filetée (28) à l'extrémité opposée à l'empreinte (29).
